(19) Europäisches Patentamt
European Patent Office
Office européen des brevets

(11) **EP 2 258 714 B1**

(12) **EUROPEAN PATENT SPECIFICATION**

(45) Date of publication and mention
of the grant of the patent:
**25.02.2015 Bulletin 2015/09**

(21) Application number: **09713305.2**

(22) Date of filing: **16.01.2009**

(51) Int Cl.:
***C07K 7/08*** (2006.01)     ***G01N 33/576*** (2006.01)
***A61K 39/29*** (2006.01)

(86) International application number:
**PCT/ES2009/000019**

(87) International publication number:
**WO 2009/103828 (27.08.2009 Gazette 2009/35)**

(54) **IMPROVED ANALYTICAL METHOD FOR THE DETECTION OF OCCULT HEPATITIS C AND CORRESPONDING DIAGNOSIS KIT**

VERBESSERTES ANALYTISCHES VERFAHREN ZUM NACHWEIS VON LATENTER HEPATITIS C UND ENTSPRECHENDES DIAGNOSEKIT

MÉTHODE ANALYTIQUE PERFECTIONNÉE PERMETTANT DE DÉTECTER UNE HÉPATITE C OCCULTE ET KIT DE DIAGNOSTIC CORRESPONDANT

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **21.02.2008 ES 200800493**

(43) Date of publication of application:
**08.12.2010 Bulletin 2010/49**

(73) Proprietor: **Fundación Para El Estudio De Las Hepatitis Virales**
**28015 Madrid (ES)**

(72) Inventors:
 • **CARREÑO GARCÍA, Vicente**
  **E-28015 Madrid (ES)**
 • **BARTOLOMÉ NEBREDA, Fernando Javier**
  **E-28015 Madrid (ES)**
 • **CASTILLO AGUILAR, Inmaculada**
  **E-28015 Madrid (ES)**
 • **QUIROGA ESTÉVEZ, Juan Antonio**
  **E-28015 Madrid (ES)**

(74) Representative: **Ungria López, Javier**
**Avda. Ramón y Cajal, 78**
**28043 Madrid (ES)**

(56) References cited:
**EP-A1- 0 489 968     US-B1- 6 667 387**

**US-B1- 7 316 905**

• **QUIROGA JUAN ANTONIO ET AL: "Patterns of immune responses to the host-encoded GOR and hepatitis C virus core-derived epitopes with relation to hepatitis C viremia, genotypes, and liver disease severity", JOURNAL OF INFECTIOUS DISEASES, vol. 173, no. 2, 1996, pages 300-305, XP002625794, ISSN: 0022-1899**
• **QUIROGA JUAN A ET AL: "Serum immunoglobulin G antibodies to the GOR autoepitope are present in patients with occult hepatitis C virus (HCV) infection despite lack of HCV-Specific antibodies", CLINICAL AND VACCINE IMMUNOLOGY, vol. 14, no. 10, October 2007 (2007-10), pages 1302-1306, XP002625795, ISSN: 1556-6811**
• **BURATTI E ET AL: "IMPROVED REACTIVITY OF HEPATITIS C VIRUS CORE PROTEIN EPITOPES IN ACONFORMATIONAL ANTIGEN-PRESENTING SYSTEM", CLINICAL AND DIAGNOSTIC LABORATORY IMMUNOLOGY, AMERICAN SOCIETY FOR MICROBIOLOGY, US, vol. 4, no. 2, 1 March 1997 (1997-03-01), pages 117-121, XP002917180, ISSN: 1071-412X**

**(Cont. next page)**

- LECHMANN M ET AL: "T- AND B-CELL RESPONSES TO DIFFERENT HEPATITIS C VIRUS ANTIGENS IN PATIENTS WITH CHRONIC HEPATITIS C INFECTION AND IN HEALTHY ANTI-HEPATITIS C VIRUS-POSITIVE BLOOD DONORS WITHOUT VIREMIA", HEPATOLOGY, WILLIAMS AND WILKINS, BALTIMORE, MD, US, vol. 24, no. 4, 1 January 1996 (1996-01-01), pages 790-795, XP001058127, ISSN: 0270-9139
- CHEN M ET AL: "Limited humoral immunity in hepatitis C virus infection", GASTROENTEROLOGY, ELSEVIER, PHILADELPHIA, PA, vol. 116, no. 1, 1 January 1999 (1999-01-01), pages 135-143, XP027312493, ISSN: 0016-5085 [retrieved on 1999-01-01]
- SALLBERG MATTI ET AL: "Immune recognition of linear antigenic regions within the hepatitis B pre-C and C-gene translation products using synthetic peptides", JOURNAL OF MEDICAL VIROLOGY, vol. 42, no. 1, 1994, pages 7-15, XP002625796, ISSN: 0146-6615
- KASHIWAKUMA T ET AL: "Detection of hepatitis C virus specific core protein in serum of patients by a sensitive fluorescence enzyme immunoassay (FEIA)", JOURNAL OF IMMUNOLOGICAL METHODS, ELSEVIER SCIENCE PUBLISHERS B.V.,AMSTERDAM, NL, vol. 190, no. 1, 28 March 1996 (1996-03-28), pages 79-89, XP004020891, ISSN: 0022-1759, DOI: DOI: 10.1016/0022-1759(95)00261-8
- QUIROGA JUAN A ET AL: "Combined hepatitis C virus (HCV) antigen-antibody detection assay does not improve diagnosis for seronegative individuals with occult HCV infection.", JOURNAL OF CLINICAL MICROBIOLOGY DEC 2006 LNKD- PUBMED:17021056, vol. 44, no. 12, December 2006 (2006-12), pages 4559-4560, XP002625797, ISSN: 0095-1137
- NIKOLAEVA L I ET AL: "Virus-specific antibody titres in different phases of hepatitis C virus infection.", JOURNAL OF VIRAL HEPATITIS NOV 2002 LNKD- PUBMED:12431205, vol. 9, no. 6, November 2002 (2002-11), pages 429-437, XP002625798, ISSN: 1352-0504
- QUIROGA J A ET AL: "752 DETECTION OF ANTIBODIES TO HCV CORE IN PATIENTS WITH SEROLOGICALLY SILENT OCCULT HCV INFECTION", JOURNAL OF HEPATOLOGY, MUNKSGAARD INTERNATIONAL PUBLISHERS, COPENHAGEN, DK, vol. 48, April 2008 (2008-04), pages S280-S281, XP026662478, ISSN: 0168-8278, DOI: DOI:10.1016/S0168-8278(08)60754-0 [retrieved on 2008-01-01]
- QUIROGA J A ET AL: "Identification of serologically silent occult hepatitis C virus infection by detecting immunoglobulin G antibody to a dominant HCV core peptide epitope", JOURNAL OF HEPATOLOGY, MUNKSGAARD INTERNATIONAL PUBLISHERS, COPENHAGEN, DK, vol. 50, no. 2, 1 February 2009 (2009-02-01), pages 256-263, XP025949516, ISSN: 0168-8278, DOI: DOI:10.1016/J.JHEP.2008.08.021 [retrieved on 2008-11-04]

SEQUENCE LISTING

<110> FUNDACIÓN PARA EL ESTUDIO DE LAS HEPATITIS VIRALES

<120> IMPROVED ANALYTICAL METHOD FOR THE DETECTION OF LATENT HEPATITIS C, USE THEREOF AND CORRESPONDING DIAGNOSIS KIT

<130> FUNDACIÓN PARA EL ESTUDIO DE LAS HEPATITIS VIRALES

<160> 1

<170> PatentIn version 3.1

<210> SEQ ID NO:1
<211> 15
<212> Peptide
<213> Synthetic peptide corresponding to the hepatitis C virus Core Protein

<220> Domain: 5-19

<400> 1

```
Pro Lys Pro Gln Arg Lys Thr Lys Arg Asn Thr Asn Arg Arg
1               5               10

Pro
15
```

<210> SEQ ID NO:2
<211> 20
<212> Peptide
<213> Synthetic peptide corresponding to the hepatitis C
virus Core Protein

<220> Domain: 21-40

<400> 2

```
Asp Val Lys Phe Pro Gly Gly Gly Gln Ile Val Gly Gly Val
1               5               10

Tyr Leu Leu Pro Arg Arg
15                  20
```

<210> SEQ ID NO:3
<211> 20
<212> Peptide
<213> Synthetic peptide corresponding to the hepatitis C
virus Core Protein

<220> Domain: 101-120

<400> 3

```
Arg Gly Ser Arg Pro Ser Trp Gly Pro Thr Asp Pro Arg Arg
1               5               10

Arg Ser Arg Asn Lys Gly
15                  20
```

**Description**

## TECHNICAL FIELD OF THE INVENTION

This invention lies within the technical field of the identification and detection of the hepatitis C virus (HCV).

More specifically, this invention provides an improved analytical method and its corresponding diagnosis kit for the detection of said virus C, which is especially useful in the identification and detection of the so-called "occult HCV infections", which cannot be detected in serum or plasma samples using current standard analytical methods.

## STATE OF THE ART

HCV is a member of the *Hepacivirus* genus within the *Flaviviridae* family of animal viruses. The HCV genome (hereinafter HCV-RNA) consists in a single-stranded RNA molecule of positive polarity that is about 9.4 kilobases (kb) long. It is assumed that HCV replication occurs via the synthesis of a negative-strand RNA chain, that is, a chain which is complementary to the molecule of genomic RNA of the virus which, in turn, acts as a template for the synthesis of new molecules of the genomic RNA. Although the liver is the main target organ for HCV infection, this virus may, potentially, infect any type of cell, tissue or organ in the human body.

Currently there are techniques that allow without difficulty diagnosing HCV infections in a patient by detecting antibodies against HCV antigens (hereinafter anti-HCV) or HCV-RNA in serum or plasma samples.

However, there is a type of HCV infection that has been recently described, **characterised in that** it is "serologically silent", wherein infected patients do not produce positive results in anti-HCV or HCV-RNA commercial detection kits in serum or plasma (hereinafter occult infection).

The general profile for this type of patients is people with abnormal results in hepatic function tests for an extended period of time, meaning abnormally high levels of hepatic enzymes. All or most of the known causes of liver disease have been assessed in these patients, which have been all ruled out based on either analytical, clinical or epidemiological data, for example: infection by hepatitis B virus (patients were negative to serum hepatitis B surface antigen and HBV-DNA), HCV (patients were negative to serum anti-HCV and HCV-RNA), chronic autoimmune hepatitis, alcoholic hepatitis, Gilbert's syndrome, biliary cirrhosis, as well as autoimmune, metabolic and genetic disorders, along with risk factors of liver damage such as consumption of alcohol, drugs, pharmaceutical products, transfusions, tattoos, body piercings, careless sexual behaviour, etc.

We are therefore dealing with a population with altered hepatic function which current analytical methods cannot explain, and the cause of which may be an occult infection by a certain form of the hepatitis C virus. Since this infection is impossible to diagnose rapidly, effectively and safely by current methods, this type of infection has two very important consequences:

- First of all, from the patient's own point of view, these patients are totally disadvantaged compared to patients whose disease can be detected in serum or plasma using current analytical methods, since specialist doctors can then study their specific condition and provide them with the most suitable therapy.
- Secondly, from the point of view of the epidemiology and dissemination of the disease resulting from the lack of knowledge on such disease both by the carriers and the specialists. Therefore, patients infected with the occult form of the hepatitis C virus carry HCV-RNA in the mononuclear cells of their peripheral blood but not in the serum, which must be considered for blood and organ donations, haemodialysis, etc., since they may be infected without this being suspected. On the other hand, knowledge of disease would allow the specialist to advise the patient on basic safety measures to prevent undesirable infection in their usual circles.

Within this technical field, researchers use immunoassays as a usual laboratory test due to the reliability of its results. Enzyme immunoassay (EIA), in particular, is a commonly used test since it is a simple, reproducible, objective method that allows processing a large amount of body fluid samples in a short time, which facilitates its implementation in less complex laboratories.

There are currently existing commercial immunoassays for determining anti-HCV in blood. These EIA detect antibodies against the structural (core and envelope) and non-structural (NS) proteins of HCV. On the other hand, the sensitivity of commercial EIAs for determining anti-HCV in blood differ according to which HCV antigens are coating the solid phase, whether these are individual or fused proteins or peptide fragments.

Commercial EIAs for the detection of anti-HCV in human serum or plasma comprise the following active ingredients:

- a solid phase (microplate wells, for example) coated with a mixture of HCV antigens: core and/or E2/NS1 and/or NS3 and/or NS4 and/or NS5. Most EIA in the market include at least the NS3 and NS4 core antigens.

- negative and positive controls for the reaction,
- sample diluent,
- washout solution,
- conjugate diluent,
- conjugate,
- substrate buffer and/or substrate solution,
- stopper solution.

The technical field of action of the commercial EIA method for detecting anti-HCV is the "classic" infection by HCV. These EIA, called EIA screening, that are used to screen a population sample of individuals that might have been in contact with the HCV, show a proven effectiveness or 99% or more regarding their specificity and an analytical sensitivity that is close to 100% in patients with classic HCV infection.

The specificity of the commercial EIA method decreases in immunocompetent groups of subjects, such as volunteer blood donors, health professionals, prison workers or military personnel. Similarly, their sensitivity is lower in immunocompromised individuals [information from the Centre for Disease Control and Prevention of Atlanta, USA, according to its publication: Morbidity and Mortality Weekly Report 2003;52(No.RR-3):1-13].

Although the immunoassay technique already has a very well established protocol, any specialist in the field should be aware that the specific determination of each specific antigen or antibody requires a certain research effort in order to find suitable working conditions and reagents for each case. As a reference of the former we provide a list of publications that use the EIA method to determine anti-HCV.

(1) Kuo G, et al. Science 1989;244:362-364.
(2) Alter HJ, et al. Lancet 1989;2:1006-1008.
(3) van der Poel CL, et al. Vox Sang 1992;62:208-212.
(4) Kleinman S, et al. Transfusion 1992;32:805-813.
(5) Okamoto H, et al. Hepatology 1992;15:180-186.
(6) Sällberg M, et al. J Clin Microbiol 1992;30:1989-1994.
(7) Sällberg M, et al. Immunol Lett 1992;33:27-33.
(8) Bresters D, et al. J Med Virol 1992;37:187-191.
(9) Sato A, et al. J Med Virol 1994;44:88-91.
(10) Sällberg M, et al. J Med Virol 1994;43:62-68.
(11) Mondelli MU, et al. J Clin Microbiol 1994;32: 2523-2527.
(12) Chen M, et al. Gastroenterology 1999;116:135-143.
(13) Toyoda H, et al. Am J Gastroenterol 1999;94:2230-2236.
(14) Laperche S, et al. J Clin Microbiol 2005;43:3877-383.
(15) Netski DM, et al. Clin Infect Dis 2005;41:667-675.
(16) Muerhoff AS, et al. J Med Virol 2008;80:411-418.

Specifically, publications (5), (6), (7), (8), (9), (10) and (16) from the list refer to the use of the core antigen in anti-HCV determination in different clinical situations of classic HCV infection. As can be observed in these publications, core protein or peptides of different sizes have been used to detect anti-core HCV antibodies.

As an example, reference (5) describes the use of a synthetic core peptide (5-23) that allows detecting anti-HCV using EIA.

Publications (6) and (7) and other related publications carry out an analysis of the antigen regions of the HCV core protein (comprising amino acids 1 to 190) to identify immunodominant regions using synthetic peptides that allow characterising the immune response in "classic" infection by HCV. Specifically: 1) a peptide is identified that comprises amino acids 1-17 of the amino terminal of the core protein; 2) the main antigenic region in the sequence of amino acids 9-16 is located; 3) anti-HCV IgG antibodies are detected by EIA using a core peptide in 83% of sera from patients with anti-HCV detectable with commercial EIA kits.

On the other hand, reference (16) and other related publications describe both the use of synthetic core protein peptides with genotype 1a and that of HCV antigens to detect anti-HCV. The use of HCV core peptides with sequences of amino acids 1-18, 10-24, 11-28 1-30, 10-43 and others produced different percentages of detection of anti-HCV by EIA. As an example, the percentage of detection of anti-HCV in the sera of patients with classic infection by HCV genotype 1 (with positive anti-HCV results by commercial EIA) using each peptide was: 78% with core 1-18; 89% with core 10-24; 85% with core 11-28; 93% with core 1-30; 100% with core 10-43. This publication does not provide data on the determination of anti-core antibodies in subjects negative to anti-HCV using commercial EIA.

The research team that has developed this invention has been using immunoassay in recent years for their determinations of HCV both in blood (serum or plasma) and in the supernatants of human cell cultures. It has used immunoassay for analytical determinations of HCV in serum or plasma samples and in samples of the supernatant of cell cultures with results described in the following references:

(1) Quiroga JA, et al. Hepatology 1991;14:38-43.
(2) Quiroga JA, et al. Clin Diag Lab Immunol 1994; 1:545-551.
(3) Quiroga JA, et al. J Infect Dis 1996;173:300-305.
(4) Martin J, et al. Cytokine 1998;10:635-644.
(5) Quiroga JA, et al. J Clin Microbiol 2006;44:4559-4560.

In particular, Quiroga JA, et al. 1996 (J Infect Dis 1996;173:300-305) discloses a HCV core peptide (amino acid 5-19) consisting of SEQ ID NO: 1 for the detection of specific antibodies, as well as an ELISA method to detect specific antibodies in serum samples from patients with chronic hepatitis C infection.

Although commercial EIA can detect anti-HCV in "classic" infection by HCV, they do not work (that is, they produce a NEGATIVE result) with the "occult" form of the disease. Commercial EIAs may not be sufficiently sensitive to detect trace amounts of anti-HCV. The reason they may not work correctly is that the mixture of antigens coating the solid phase may interfere and prevent the detection of trace amounts of anti-HCV.

In order to solve these problems we have tried some modifications or alternative applications.

Thus, we determined the presence of anti-HCV using the MONOLISA® HCV Ag-Ab ULTRA assay (Bio-Rad Laboratories, Marnes-la-Coquette, France) in serum samples from patients diagnosed with occult infection by HCV (negative anti-HCV results with commercial EIAs: Ortho HCV 3.0 ELISA, Ortho Diagnostic Systems, Raritan, NJ, USA; and INNOTEST HCV Ab IV, Innogenetics, Gante, Belgium). The MONOLISA® HCV Ag-Ab ULTRA assay was only capable of detecting the presence of anti-VHC in the serum of 1 patient with occult infection by HCV [Quiroga JA, et al. J Clin Microbiol 2006;44:4559-4560]. These data show that the alternatives for anti-HCV detection do not work.

From the above it can be deduced that there is a need for a fast, safe and effective method to determine the existence of occult HCV infection in a subject suspected of infection. The gold standard method for identifying occult HCV infection is HCV-RNA detection from a biopsy of liver tissue.

In this regard, Spanish Patent No. 200303050 describes an analytical method based on in situ hybridisation that detects HCV-RNA in liver tissue, and therefore identifies occult HCV infection that cannot be detected in serum or plasma samples using standard analytical methods.

Nevertheless, biopsies are invasive methods that cannot always be performed since they involve risks for the patients. Therefore, a simple and sensitive method is required to diagnose occult HCV infection from blood samples.

Quiroga J. A. et al., 2007 [Quiroga JA, et al. Clinical and Vaccine Immunology 2007;14(10):1302-1306] discloses a method for the detection of occult hepatitis C virus (HCV) infection by screening sera for anti-GOR immunoglobulin G. This document describes the implementation of anti-GOR IgG testing for the detection of occult hepatitis C virus, obtaining values of specificity and sensitivity of 100% and 20% respectively. However, even after implementation of anti-GOR IgG testing and in view of the 20% sensitivity obtained, the majority of patients would still need a liver biopsy for the accurate diagnosis of occult HCV infection, which cannot always be performed since they are invasive and involve risks for the patients.

Researchers have intensified the search for the optimal parameters of the immunoassay protocol (e.g., method of preparation of the reaction matrix, pretreatment of samples prior to the immunochemistry reaction, type and strength of the conjugate antiserum, time necessary for the immunochemistry reaction to occur, etc.) for diagnosing a potential occult HCV infection.

## DESCRIPTION OF THE INVENTION

This invention, as described in the title, relates to a improved analytical method for the detection of occult hepatitis C, the applications of this analytical method and its corresponding diagnostic kit.

Specifically, it relates to a method for the detection of occult hepatitis C according to claim 1 which comprises performing a screening immunoassay test. A preferred embodiment of the above is an immunoassay chosen from ELISA, radioimmunoassay, or a fluorescence, chemiluminescence or bioluminescence immunoassay.

Said screening immunoassay for detecting occult hepatitis C uses serum or plasma samples from peripheral blood or from organs or fluids produced or secreted by isolated cells, or secretions from tissues or organs, or blood or fluids from organs.

The method comprises performing a screening immunoassay for detecting occult hepatitis C wherein the HCV reagent is the synthetic peptide of the HCV core protein consisting of SEQ.ID.NO:1.

A particular embodiment uses, as well as the peptide consisting of SEQ.ID.NO:1, an additional peptide consisting of sequence SEQ.ID.NO:2.

A particular embodiment uses, as well as the peptide consisting of SEQ.ID.NO:1, an additional peptide consisting of sequence SEQ.ID.NO:3.

A particular embodiment uses, as well as the peptide consisting of SEQ.ID.NO:1, the two additional peptides consisting of sequences SEQ.ID.NO:2 and SEQ.ID.NO:3.

The preferred immunoassay of the invention is an ELISA using as an antigen the peptide consisting of SEQ.ID.NO:1, the combination of the two peptides of sequences SEQ.ID.NO:1 and SEQ.ID.NO:2, the combination of the two peptides of sequences SEQ.ID.NO:1 and SEQ.ID.NO:3 and the combination of the three peptides of sequences: SEQ.ID.NO:1, SEQ.ID.NO:2 and SEQ.ID.NO:3.

On the other hand, in other assays carried out by the researchers it was observed that other peptides of the core protein with a sequence of 20 amino acids other than that consisting of SEQ.ID.NO:1 also showed antigen functionality. However, the sensitivity of anti-HCV detection was lower than using the 15 amino acid peptide of the present invention.

According to an aspect of the disclosure in which the screening immunoassay uses the peptide consisting of sequence SEQ.ID.NO:2 as an antigen, anti-HCV IgG core is detected by EIA in the serum of 100% of patients with classic HCV infection of genotypes 1, 3 and 4 (with anti-HCV positive results using commercial EIA tests). Anti-HCV IgG core in the serum of 15% of patients with occult HCV infection is detected according to this aspect of the disclosure.

According to another aspect of the disclosure in which the screening immunoassay uses the peptide consisting of sequence SEQ.ID.NO:3 as an antigen, anti-HCV IgG core is detected by EIA in the serum of 75% of patients with classic HCV infection of genotypes 1, 3 and 4 (with anti-HCV positive results using commercial EIA tests). According to this aspect of the disclosure anti-HCV IgG core in the serum of 25% of patients with occult HCV infection is detected.

In one embodiment of the invention using the peptides consisting of sequences SEQ.ID.NO:1, SEQ.ID.NO:2 and SEQ.ID.NO:3 detects anti-HCV IgG core by EIA in the serum of 100% of the patients with classic HCV infection with genotype 1, 3 or 4 (with positive anti-HCV results using commercial EIA tests). The novel application also detects anti-HCV IgG core in the serum of 50% of patients with occult HCV infection with genotype 1 (that is, with negative anti-HCV results using commercial EIA tests). It can therefore be said that the analytical method object of this invention increases the sensitivity of existing EIA tests, improves serological diagnosis and may be useful for tracking occult HCV infection.

Such that another preferred embodiment of the invention uses as an antigen for the screening immunoassay a peptide consisting of sequence SEQ.ID.NO:1 and one or two of the peptides consisting of sequences: SEQ.ID.NO:2 and SEQ.ID.NO:3.

The method of the invention wherein the peptide consisting of sequence SEQ.ID.NO:1 is used, detects by EIA anti-HCV IgG core in the serum of 98% of patients with classic HCV infection with genotype 1 (with positive anti-HCV results using commercial EIA tests). The novel application is that it also detects anti-HCV IgG core in the serum of 40% of patients with occult HCV infection (that is, with negative anti-HCV results using commercial EIA tests). We can therefore say that the analytical method object of this invention increases the sensitivity of existing EIA tests, improves serological diagnosis and may be useful for tracing occult HCV infection.

Therefore, in other populations with potential risk of occult HCV infection anti-HCV IgG core is detected in the serum of 27% of the relatives of patients with occult HCV infection and 35% of relatives of patients with classic HCV infection who gave negative anti-HCV results using commercial EIA tests. That is, compared to commercial anti-HCV screening methods, the measurement of anti-HCV IgG core allows determining exposure to HCV, and its transmission between relatives of HCV-infected patients.

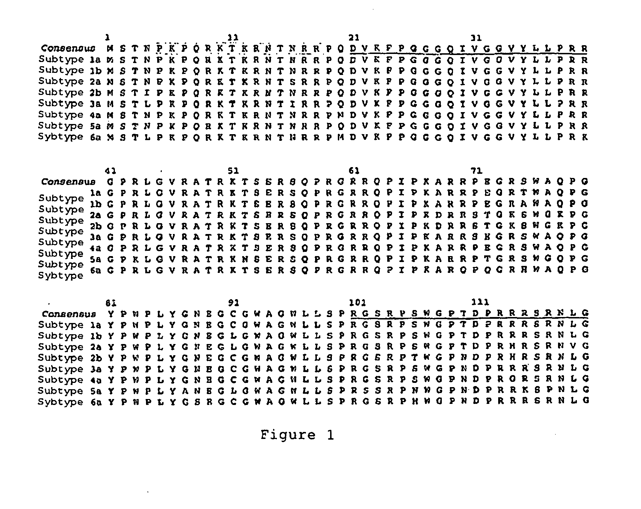

On the other hand, although no occult HCV infection has been described with a genotype other than genotype 1b, the method of the invention is capable of detecting anti-HCV IgG core antibodies in the serum or plasma of patients with classic HCV infection with genotype 1 (either 1b or 1a), as well as cases with genotypes other than genotype 1. The sequence with sequence tag SEQ.ID.NO:1 has some advantages regarding other longer sequences: When omitting positions 4 and 20 of the peptide's amino acid sequence, some variable positions are also excluded that are immediately before and after, present in genotypes 2b, 3, 4 and 6 (see Figure 1), and therefore SEQ.ID.NO:1 is preserved amongst the different HCV genotypes 1 to 6 (see the Consensus line in Figure 1). The genotype dependence of anti-HCV IgG core detection is eliminated when using SEQ.ID.NO:1 as the HCV reagent (described in example 2), (as recognised by the authors of the publication of reference No. 16, especially due to position 4).

On the other hand, in other experiments carried out we observed that shorter peptides, with sequences of between 6 and 10 amino acids from the amine end of the core protein, showed antigen functionality. However, the sensitivity of anti-HCV detection was lower than using the 15 amino acid peptide of the invention.

According to the options found in the state of the art, this immunoassay can use an antiserum labelled with an isotopic or non-isotopic tracer. Amongst the latter, a preferred embodiment of the invention comprises the use of a fluorophore, a chromophore, an enzyme or any other molecule that produces a conjugate that is suitable for immunoassay tests. An even more preferred embodiment uses an anti-human IgG antibody conjugated with the horseradish peroxidase enzyme (anti-IgG-HRP).

Also described herein is a diagnostic kit for the implementation of said analytical method for the detection of occult hepatitis C. Specifically, said kit comprises the essential components of the screening immunoassay to be performed, such as a Reaction matrix, Reagent, HCV, Stopping agent, Sample diluent, Washout solution, Conjugate solution and Reagents for developing the immunochemistry reaction. According to the present invention, the kit comprises the following components:

> i) a solid-phase matrix,
> ii) a HCV reagent, wherein the HCV reagent is a synthetic peptide of the HCV core protein consisting of SEQ.ID.NO:1, and one or two of the peptides consisting of sequences: SEQ.ID.NO:2 and SEQ.ID.NO:3,
> iii) a blocking agent,
> iv) a washout solution,
> v) a conjugate solution
> vi) a developing solution, and
> vii) a reaction stopper solution.

Specifically, the screening immunoassay that can be performed with this kit is chosen from an ELISA, a radioimmunoassay, a fluorescence, chemiluminescence or bioluminescence immunoassay.

Said screening immunoassay performed with the kit for detecting occult hepatitis C uses serum or plasma samples from peripheral blood or from organs, from fluids produced or secreted by isolated cells, or from secretions of tissues or organs, or from blood or fluids from organs.

A particular embodiment of this diagnostic kit uses the peptide consisting of SEQ.ID.NO:1, and an additional peptide consisting of sequence SEQ.ID.NO:2.

An additional particular embodiment of this diagnostic kit uses, as well as the peptide consisting of SEQ.ID.NO:1, an additional peptide consisting of sequence SEQ.ID.NO:3.

An additional particular embodiment of this diagnostic kit uses, as well as the peptide consisting of SEQ.ID.NO:1, the two additional peptides consisting of sequences SEQ.ID.NO:2 and SEQ.ID.NO:3.

The analytical method of this invention based on immunoassays can be summarised in the diagram as follows:

*Diagram of the method*

Preparation of the reagent and the reaction matrix
↓

Pre-treatment of the sample

↓

Antigen-antibody immunochemistry reaction
↓

Washout
↓

Reaction detection
↓

Washout
↓

Reaction development
↓

Reaction measurement

Since the analytical method described herein is especially intended for the detection of anti-HCV in occult infection, it is necessary to optimise the different stages of the general method for this specific case, in order to achieve the improved analytical method object of the present invention. Thus, the method for the detection of occult hepatitis C comprises:

a) incubating a solid-phase matrix with a HCV reagent, wherein the HCV reagent is a synthetic peptide of the HCV core protein consisting of SEQ.ID.NO: 1, and optionally also one or two of the peptides consisting of sequences SEQ.ID.NO:2 and SEQ ID.NO:3, for the preparation of the reagent and the reaction matrix;

b) pre-treating the serum or plasma sample by a dilution and an incubation the purpose of which is to adsorb the sample components in order to eliminate the non-specific antigen-antibody bonds;

c) adding the pre-treated sample obtained in step b) to the solid-phase matrix obtained in step a) and incubating the same for the antigen-antibody immunochemistry reaction;

d) washout and reaction detection;

e) washout and reaction development; and

f) reaction measurement.

Solid-phase immunoassays are based on an immunochemistry reaction which takes place in a reaction matrix or support that can be a microplate divided into small cells or wells for individual samples, tubes, spheres, strips or combinations thereof, of an organic material such as polystyrene, polyethylene, polyvinyl, polycarbonate, cellulose, etc.

The target molecules of the method are specific anti-HCV antibodies of the hepatitis C virus structural core protein. The samples containing these target molecules are either serum or plasma samples taken from patients in whom no anti-HCV or HCV-RNA antibodies are detected in serum or plasma using available commercial methods, but who have sustained abnormal values of liver enzymes in their blood.

The samples are usually obtained from blood extracted from the patients.

The following examples illustrate the method of the invention without meaning to limit it in any way.

## Brief description of the drawing

Figure 1 shows the alignment of the HCV core amino acid sequences from position 1 to 120. The consensus sequence between the 6 main sequences is indicated by the Consensus line; the region corresponding to SEQ.ID.NO:1 is shaded, whereas regions corresponding to sequences SEQ.ID.NO:2 and SEQ.ID.NO:3 are underlined. The GenBank accession number for the sequences shown is: AF009606, subtype 1a; AF165064, subtype 1b; AB047639, subtype 2a; AY232749, subtype 2b; D17763, subtype 3a; Y11604, subtype 4a; Y13184, subtype 5a; Y12083, subtype 6a.

## EXAMPLES

### Example 1. Obtaining the samples.

The serum or plasma samples are obtained from 10 ml of venous blood extracted with a suitable device and placed in a glass tube of the Vacutainer® type (Beckton-Dikinson, Plymouth, UK) with gel on the bottom, designed to obtain serum, or containing anticoagulant (170 IU of lithium heparin) to obtain plasma. The blood is then centrifuged for 20 minutes at 1,200 x g at ambient temperature (between 20 and 25°C) in a 1.0R Heraeus Megafuge centrifuge (Heraeus, Osterode, Germany). The upper layer containing the serum or plasma is separated and approximately 1 ml of this is placed in a polystyrene tube with a cap, suitable for storing serum or plasma at -20°C until use.

### Example 2. Obtaining the HCV reagent.

The HCV reagent used in the method object of the invention is a synthetic pentadecapeptide with the sequence of SEQ.ID.NO:1, comprising amino acids 5 to 19 of the N-terminal end of the HCV core protein (hereinafter, core peptide 5-19). Core peptide 5-19 was synthesised by GeneScript Corporation (Scotch Plains, NJ, USA). The lyophilised core peptide 5-19 contains 7 mg of protein with a purity of 93.8% according to analyses performed by high performance liquid chromatography and mass spectrometry. The lyophilised core peptide 5-19 is dissolved in ultrapure H2O (Milli-Q; Millipore, Molsheim, France) to obtain a concentration of 1 mg/ml; it is divided into 220 $\mu$l aliquots in sterile 1 ml polypropylene tubes with caps and stored at a temperature of -20°C until use.

### Example 3. Preparation of the reaction matrix (solid phase).

The solid phase consists of a flat bottom, sterile, 96-well EIA polystyrene microtitre plate, without caps (Costar, Cambridge, MA, USA). To prepare the reaction matrix, thaw an aliquot of the core peptide 5-19 of those described in Example 2 to 4°C. Prepare a reagent solution with a concentration of 10 μg/ml by mixing 110 μl of reconstituted core peptide 5-19 with 10.89 ml of 0.1 M buffered sodium carbonate solution at pH 9.6, pre-cooled to 4°C. Place 100 μl of the 10 μg/ml core peptide 5-19 solution in each well and incubate for 18 hours at a temperature of 4°C without stirring in an S-1000-2 refrigerator (Azkoyen, Madrid, Spain) At the end of the incubation period wash each well twice with 200 μl of washout solution made from 0.1 M sodium phosphate buffer at pH 7.4 to which 0.05% of polyoxyethylene-(20)-sorbitan monolaurate (Tween-20®/ Sigma Chemical Co., St. Louis, MO, USA) has been added. When the last washout has finished, remove the excess washout solution by turning the plate upside down over absorbent paper. Then add 150 μl of the blocking agent made from 0.1 M buffered sodium and potassium phosphate solution at pH 7.4, supplemented with 10% of foetal bovine serum (Sera Laboratories International Ltd., West Sussex, UK), inactivated by heating at 56°C for 30 minutes, to which 0.05% of Tween-20® is then added, incubating the whole at 37°C for 60 minutes in a Heraeus I-42 incubator. Withdraw the excess blocking agent at the end of the incubation period by turning the plate upside down. Wash each well twice with 200 μl of washout solution. When the last washout has finished, remove the excess washout solution by turning the plate upside down over absorbent paper. At the end of this stage the reaction matrix will be ready for incubation with the samples.

*Variant 1 of Example 3.*

This first option consists in performing simultaneous determination using peptides consisting of SEQ.ID.NO:1, SEQ.ID.NO:2 and SEQ.ID.NO:3 separately to prepare the reaction matrix (Example 3 of the application). Such that "100 μl of the 10 μg/ml core peptide 5-19 solution are placed in each well and incubated for 18 hours at a temperature of 4°C..."; or "100 μl of the 10 μg/ml core peptide 21-40 solution are placed in each well and incubated for 18 hours at a temperature of 4°C..."; or "100 μl of the 10 μg/ml core peptide 101-120 solution are placed in each well and incubated for 18 hours at a temperature of 4°C...". The plate wells can be coated with only one of the peptides, or using two peptides and coating half the plate wells with each peptide, or using the three peptides and coating a third of the plate wells with each peptide. The rest of the method is the same."

*Variant 2 of Example 3.*

This second option consists in carrying out the invention by combining the peptides with sequence consisting of SEQ.ID.NO:1, SEQ.ID.NO:2 and SEQ.ID.NO:3 to prepare the reaction matrix (Example 3 of the application). Such that 100 μl of a mixed solution of the two peptides with sequences corresponding to SEQ.ID.NO:1 and SEQ.ID.NO:2, a mixed solution of SEQ.ID.NO:1 and SEQ.ID.NO:3, or a mixed solution of SEQ.ID.NO:1, SEQ.ID.NO:2 and SEQ.ID.NO:3 are placed in the plate wells, that is, for example, "100 μl of a mixed solution of the 10 μg/ml core peptide 5-19 and the 10 μg/ml core peptide 101-120 solution are placed in each well and incubated for 18 hours at a temperature of 4°C ...", etc. The rest of the method is the same.

### Example 4. Pre-treatment of the sample.

The serum or plasma sample is thawed from -20°C to room temperature (between 20°C and 25°C), by repeatedly inverting the tube to homogenise its contents before pretreatment. This pretreatment is a crucial stage in the method object of the invention, a step that is not specified in the protocols of most commercial EIA tests. This consists in an incubation the purpose of which is to adsorb the sample components in order to eliminate the non-specific antigen-antibody bonds that might conceal trace amounts of anti-HCV. 50 μl of serum or plasma sample are placed in a 1.5 ml polypropylene microtube (Sarsted, Nümbrecht, Germany) and 450 μl are added of a 0.1 M buffered sodium and potassium phosphate solution at pH 7.4 supplemented with 10% of foetal bovine serum (Sera Laboratories International Ltd.) and 0.05% of Tween-20® (Sigma). The mixture is incubated at 37°C for 1 h in a Thermomixer 5436 incubator (Eppendorf, Hamburg, Germany).

### Example 4bis. Pre-treatment of the sample for blocking.

The serum or plasma sample is thawed from -20°C to ambient temperature (between 20 and 25°C), repeatedly turning the tube to homogenise its contents before pretreatment. This sample pretreatment variant demonstrates the detection specificity for anti-HCV according to the method object of the invention. It consists in an incubation the purpose of which is to mask the specific anti-HCV antibodies of the sample using the non-specific antibody-antigen peptide bond in order to mask the specific anti-HCV antibodies in the sample and therefore block their binding to the HCV reagent by the immunochemistry reaction described in Example 5. 50 μl of serum or plasma sample are placed in a 1.5 ml polypropylene microtube (Sarsted, Nümbrecht, Germany) and 450 μl are added of a 0.1 M buffered sodium and potassium phosphate solution at pH 7.4 supplemented with 10% of foetal bovine serum (Sera Laboratories International Ltd.) to which 0.05% of Tween-20® (Sigma) are added that do not contain ("no blocking") or contain ("blocking") sufficient amounts (between 10 and 1000 $\mu$g/ml) of the HCV reagent described in Example 2 (synthetic pentadecapeptide with the sequence of SEQ.ID.NO:1). Another peptide reagent of the HCV core protein (amino acids 21-40 with the sequence shown in Figure 1) that is irrelevant with respect to the sequence described in SEQ.ID.NO:1 is used under the same conditions as a control. The mixture is incubated at 37°C for 1 h in a Thermomixer 5436 incubator (Eppendorf, Hamburg, Germany).

*Example 5: The antigen-antibody reaction in immunochemistry.*

Samples pretreated according to the previous example (100 $\mu$l) are added to the wells of reaction matrix; the plate is covered with a transparent sheet ("Plate Sealer", Perkin Elmer - Wallac, Turku, Finland) and incubated for 1 hour at 37°C in a Heraeus I-42 incubator. When the incubation has finished withdraw the transparent sheet and wash each well 5 times with 200 $\mu$l of washout solution. When the last washout has finished, remove the excess washout solution by turning the plate upside down over absorbent paper.

*Example 6. Preparation of the conjugated antiserum.*

The conjugated antiserum used in the method object of the invention is a polyclonal anti-human immunoglobulin G (IgG) rabbit antiserum obtained against IgG isolated from normal human sera which is conjugated with the horseradish peroxidase enzyme (anti-IgG-HRP; DakoCytomation A/S, Glostrup, Denmark). The conjugated antiserum is supplied as a fluid in a buffered solution of 0.05 mmol/l Tris-HCl, 15 mmol/l NaN$_3$ at pH 7.2 at a concentration of 1 mg/mL. It is kept at 4°C until use. To prepare the conjugated antisera solution 11 $\mu$l of anti-IgG-HRP and 11 ml of blocking agent solution (described in Example 3) are mixed, obtaining a 1:1000 dilution that is optimal for this type of EIA (Harlow D & Lane D. Antibodies: A laboratory manual. Cold Spring Harbor Laboratory, New York; 1988: pp592). 100 $\mu$l of the conjugated antisera solution are added to each well of the reaction in order to detect the antigen-antibody immunochemistry reaction; the plate is covered with a transparent sheet ("Plate Sealer", Perkin Elmer - Wallac) and incubated for 1 hour at 37°C in a Heraeus I-42 incubator. When the incubation has finished withdraw the transparent sheet and wash each well 5 times with 200 $\mu$l of washout solution. When the last washout has finished, remove the excess washout solution by turning the plate upside down over absorbent paper.

*Example 7. Developer to detect the reaction.*

The method object of the invention uses the 2,2'-azinobis-(3-ethylbenzothiazoline-6-sulphonic acid) diammonium salt as a developing solution (ABTS; Pierce, Rockford, IL, USA). ABTS is a chromogenic substrate of horseradish peroxidase that gives rise to a green coloured product when oxidized. ABTS is supplied as a ready-to-use liquid solution (which does not require subsequent preparation) and is stored at 4°C until use. The ABTS solution must be brought to room temperature before use (between 20°C and 25°C). The ABTS substrate (100 $\mu$l/well) is added to the microplate wells and incubated for 30 minutes in the dark (for which the entire plate is covered, for example, with a sheet of aluminium foil) with gentle stirring in an Atom 85 stirrer (Atom, Barcelona, Spain). Optionally, a 1% n-sodium dodecyl sulphate can be used as a reaction stopper solution (Calbiochem - Biosciences Inc., La Jolla, CA, USA) (100 $\mu$l/well). The reaction is measured in a Whittaker Microplate Reader 2001 (Anthos Labtec Instruments, Salzburg, Austria) at a wavelength of 405 nm with a reference wavelength of 620 nm. The intensity of the green colour depends on the amount of anti-HCV present in the sample analysed.

## EXAMPLES OF TRIALS IN STANDARDISED PATIENTS

Example 8. Patients with occult HCV infection

Determining the cut-off value:

The samples from patients diagnosed with HCV infection are obtained following the method described in Example 1. The sample is then analysed to detect the presence of anti-HCV using ELISA with the method object of the invention. To do this, the reaction matrix is prepared following the description of example 3 using the reagent of example 2.

The sample (serum in this case) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

- The negative controls provide mean values of 0.047, 0.055 and 0.050 absorbance units at 405/620 nm; the mean value for the 3 negative controls is 0.051 absorbance units at 405/620 and the calculated standard deviation is 0.004.
- The mean value for the positive control is 0.789 absorbance units at 405/620 nm.
- The cut-off value between the NEGATIVE and POSITIVE results is determined (test cut-off value) as the addition of the mean value of the 3 negative controls plus five times the standard deviation for the mean value.
- The cut-off value for the test is 0.071 absorbance units at 405/620 nm.

*Patient No. 1*

Patient (female) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1 HCV-RNA, and therefore occult HCV infection is diagnosed and liver lesion is detected (liver cirrhosis with inflammatory activity).

- The serum from Patient No. 1 provides absorbance values of 0.166 and 0.172 units at 405/620 nm; the mean value for the serum from Patient No.1 is 0.169 absorbance units at 405/620 nm. The mean value for the sample from Patient No. 1 is greater than the cut-off value for the test, and is therefore assigned a POSITIVE result: the serum analysed contains anti-HCV antibodies.

*Patient No. 2*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and a liver lesion is detected (periportal chronic hepatitis with stage 2 fibrosis).

- The serum from Patient No. 2 provides absorbance values of 0,102 and 0,107 units at 405/620 nm; the mean value for the serum from Patient No. 1 is 0.105 absorbance units at 405/620 nm. The mean value for the sample from Patient No. 2 is greater than the cut-off value for the test, and is therefore assigned a POSITIVE result: the serum analysed contains anti-HCV antibodies.

Examples 8bis. Patients with occult HCV infection

*Specificity of the anti-HCV detection:*

The samples from patients diagnosed with HCV infection are obtained following the method described in Example 1. The sample is then analysed to detect the presence of anti-HCV using ELISA with the method object of the invention. To do this, the reaction matrix is prepared following the description of example 3 using the reagent of example 2.
The sample (serum in this case) is pre-treated as described in Example 4bis and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5.
The following results are obtained when measuring the reaction:

- The negative controls provide mean values of 0.054, 0.069 and 0.063 absorbance units at 405/620 nm; the mean value for the 3 negative controls is 0.061 absorbance units at 405/620 and the calculated standard deviation is 0.006.
- The mean value for the positive control is 1.391 absorbance units at 405/620 nm.
- The cut-off value between the NEGATIVE and POSITIVE results is determined (test cut-off value) as the addition of the mean value of the 3 negative controls plus five times the standard deviation for the mean value.

The cut-off value for the test is 0.091 absorbance units at 405/620 nm.
The reaction is considered specific if it blocks anti-HCV detection. That is, the absorbance values measured by sample pre-treatment in the presence of the HCV reagent corresponding to SEQ.ID.NO:1 are at least 50% less than when the pretreatment is performed without the HCV reagent. Moreover, the reaction is also considered specific if it does not block anti-HCV detection when pre-treating the sample in the presence of the control reagent described in example 4bis; the absorbance values measured decrease less than 10% compared to the mean values measured when pretreating without the HCV reagent. The percentage of anti-HCV detection blocking will be calculated according to following formula:

$$\% \text{ blocking} = 100 - 100 \times$$

$$\frac{(\text{absorbance of sample with HCV reagent}) - (\text{absorbance of negative control with HCV reagent})}{(\text{absorbance of sample without HCV reagent}) - (\text{absorbance of negative control without HCV reagent})}$$

*Patient No. 3*

Patient (female) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1 HCV-RNA, and therefore occult HCV infection is diagnosed and liver lesion is detected (liver cirrhosis with inflammatory activity).

- The serum from Patient No. 3 with sample pretreatment without HCV reagent provides a mean value of 0.125 absorbance units at 405/620 nm. The mean value for the sample from Patient No. 3 is greater than the cut-off value for the test, and is therefore assigned a POSITIVE result: the serum analysed contains anti-HCV antibodies.
- The serum from Patient No. 3 with sample pretreatment with 100 $\mu$g of HCV reagent (core peptide 5-19) provides a mean value of 0.072 absorbance units at 405/620 nm. By applying the formula described above we obtain an 82% block of the anti-HCV reaction, and it is therefore assigned a result of IS NOT BLOCKED: the anti-HCV reaction is considered specific.
- The serum from Patient No. 3 with sample pretreatment with 100 $\mu$g of control HCV reagent (core peptide 21-40) provides a mean value of 0.156 absorbance units at 405/620 nm. By applying the formula described above we obtain a 0% block of the anti-HCV reaction, and it is therefore assigned a result of IS NOT BLOCKED: the anti-HCV reaction is considered specific.

*Patient No. 4:*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and reactive changes are detected in the liver (minimum lesion).

- The serum from Patient No. 4 with sample pretreatment without HCV reagent provides a mean value of 0.150 absorbance units at 405/620 nm. The mean value for the sample from Patient No. 4 is greater than the cut-off value for the test, and is therefore assigned a POSITIVE result: the serum analysed contains anti-HCV antibodies.
- The serum from Patient No. 4 with sample pretreatment with 100 $\mu$g of HCV reagent (core peptide 5-19) provides a mean value of 0.060 absorbance units at 405/620 nm. By applying the formula described above we obtain an 97% block of the anti-HCV reaction, and it is therefore assigned a result of IS NOT BLOCKED: the anti-HCV reaction is considered specific.
- The serum from Patient No. 4 with sample pretreatment with 100 $\mu$g of control HCV reagent (core peptide 21-40) provides a mean value of 0.174 absorbance units at 405/620 nm. By applying the formula described above we obtain a 0% block of the anti-HCV reaction, and it is therefore assigned a result of IS NOT BLOCKED: the anti-HCV reaction is considered specific.

*Patient No. 5:*

Patient (female) who presents at the clinics due to abnormal values of hepatic enzymes. Classic HCV infection is diagnosed (anti-HCV and HCV-RNA detectable in serum using commercial methods). The liver biopsy proves the presence of genotype 1b HCV-RNA and liver lesion (active chronic hepatitis with stage 1 liver fibrosis).

- The serum from Patient No. 5 with sample pretreatment without HCV reagent provides a mean value of 1.391 absorbance units at 405/620 nm. The mean value for the sample from Patient No. 5 is greater than the cut-off value for the test, and is therefore assigned a POSITIVE result: the serum analysed contains anti-HCV antibodies.
- The serum from Patient No. 5 with sample pretreatment with 100 μg of HCV reagent (core peptide 5-19) provides a mean value of 0.146 absorbance units at 405/620 nm. By applying the formula described above we obtain an 93% block of the anti-HCV reaction, and it is therefore assigned a result of IS NOT BLOCKED: the anti-HCV reaction is considered specific.
- The serum from Patient No. 5 with sample pretreatment with 100 μg of control HCV reagent (core peptide 21-40) provides a mean value of 1.424 absorbance units at 405/620 nm. By applying the formula described above we obtain a 0% block of the anti-HCV reaction, and it is therefore assigned a result of IS NOT BLOCKED: the anti-HCV reaction is considered specific.

*Titration of anti-HCV:*

This allows measuring the amount of anti-HCV antibody present in the sample to be analysed. This measurement can be related to clinical, histopathological, virological or other characteristics and with the progression of HCV infection, either occult or classic, and the follow-up following antiviral treatment. The samples from patients diagnosed with HCV infection are obtained following the method described in Example 1. The sample is then analysed to detect the presence of anti-HCV using ELISA with the method object of the invention. To do this, the reaction matrix is prepared following the description of example 3 using the reagent of example 2. The sample is pretreated (serum in this case) as in Example 4. The pretreated sample, which is diluted to 1:10, is diluted in a twofold dilution series, i.e., 1:20, 1:40, 1:80, etc.; the immunochemistry reaction is performed as in Example 5 and the presence of anti-HCV is detected by ELISA, following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

*Patient No. 6:*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and steatosis is observed with minimum inflammatory changes in the liver. The following average results are obtained in absorbance units at 405/620 nm when measuring the reaction:

| Dilution: | 1:10 | 1:20 | 1:40 | 1:80 | 1:160 | 1:320 | 1:640 | 1:1280 | 1:2560 | 1:5120 |
|---|---|---|---|---|---|---|---|---|---|---|
| negative c. | 0.080 | 0.074 | 0.066 | 0.063 | 0.061 | 0.054 | 0.049 | 0.048 | 0.047 | 0.045 |
| cut-off v | 0.087 | 0.077 | 0.068 | 0.066 | 0.064 | 0.057 | 0.050 | 0.050 | 0.049 | 0.047 |
| Pat. No.6 | 0.396 | 0.263 | 0.151 | 0.086 | 0.069 | 0.056 | 0.046 | 0.040 | 0.035 | 0.035 |
| Results: | POS | POS | POS | POS | POS | NEG | NEG | NEG | NEG | NEG |
| (POS: POSITIVE result; NEG: NEGATIVE result) | | | | | | | | | | |

The serum analysed contains anti-HCV antibodies at a concentration of 1:160.

*Patient No. 7:*

Patient (female) who presents at the clinics due to abnormal values of hepatic enzymes. Classic HCV infection is diagnosed (anti-HCV and HCV-RNA detectable in serum using commercial methods). The liver biopsy proves the presence of genotype 1a HCV-RNA and liver lesion (periportal hepatitis with septal fibrosis). The following average results are obtained in absorbance units at 405/620 nm when measuring the reaction:

| Dilution: | 1:10 | 1:20 | 1:40 | 1:80 | 1:160 | 1:320 | 1:640 | 1:1280 | 1:2560 | 1:5120 |
|---|---|---|---|---|---|---|---|---|---|---|
| negative c. | 0.080 | 0.074 | 0.066 | 0.063 | 0.061 | 0.054 | 0.049 | 0.048 | 0.047 | 0.045 |
| cut-off v | 0.087 | 0.077 | 0.068 | 0.066 | 0.064 | 0.057 | 0.050 | 0.050 | 0.049 | 0.047 |

(continued)

| Pat. No.7 | 1.517 | 1.302 | 1.078 | 0.757 | 0.559 | 0.348 | 0.216 | 0.151 | 0.063 | 0.045 |
|---|---|---|---|---|---|---|---|---|---|---|
| Results: | POS | POS | POS | POS | POS | POS | POS | POS | POS | NEG |
| (POS: POSITIVE result; NEG: NEGATIVE result) | | | | | | | | | | |

The serum analysed contains anti-HCV antibodies at a concentration of 1:2560.

*Anti-HCV isotype:*

This allows knowing the type and isotype of the immunoglobulin (IG) of the anti-HCV antibody (type: IgA, IgG, IgM; IgG isotype: IgG1, IgG2, etc.) present in the sample to be analysed. This measurement can be related to clinical, histopathological, virological or other characteristics and with the progression of HCV infection, either occult or classic, and the follow-up following antiviral treatment. The samples from patients diagnosed with HCV infection are obtained following the method described in Example 1. The sample is then analysed to detect the presence of anti-HCV using ELISA with the method object of the invention. To do this, the reaction matrix is prepared following the description of example 3 using the reagent of example 2. The sample (in this case serum) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The reagent of example 6 (anti-IgG-HRP) is replaced by a specific reagent of the same type (e.g.: anti-IgM-HRP) or isotype (e.g.: anti-IgG1-HRP) as the antibody to be detected. The following results are obtained when measuring the reaction:

*Patient No. 8:*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and minimal liver lesions are observed. The following average results are obtained in absorbance units at 405/620 nm when measuring the reaction:

| Ig type/ isotype: | IgG | IgG1 | IgG3 |
|---|---|---|---|
| reagent: | anti-IgG-HRP | anti-IgG1-HRP | anti-IgG3-HRP |
| dilution: | 1:1000 | 1:1000 | 1:10000 |
| negative c.: | 0.065 | 0.034 | 0.030 |
| cut-off value: | 0.089 | 0.047 | 0.042 |
| Pat. No.8: | 0.325 | 0.069 | 0.029 |
| Results: | POS | POS | NEG |
| (POS: POSITIVE result; NEG: NEGATIVE result) | | | |

The serum analysed contains anti-HCV antibodies of type IgG and isotype IgG1.

*Patient No. 9:*

Patient (female) who presents at the clinics due to abnormal values of hepatic enzymes. Classic HCV infection is diagnosed (anti-HCV and HCV-RNA detectable in serum using commercial methods). The liver biopsy proves the presence of genotype 1b HCV-RNA and liver lesion (active chronic hepatitis with stage 1 liver fibrosis). The following average results are obtained in absorbance units at 405/620 nm when measuring the reaction:

| Ig type/ isotype: | IgG | IgG1 | IgG3 |
|---|---|---|---|
| reagent : | anti-IgG-HRP | anti-IgG1-HRP | anti-IgG3-HRP |
| dilution: | 1:1000 | 1:1000 | 1:10000 |
| negative c.: | 0.055 | 0.034 | 0.030 |
| cut-off value: | 0.089 | 0.047 | 0.042 |

(continued)

| Ig type/ isotype: | IgG | IgG1 | IgG3 |
|---|---|---|---|
| Pat. No.9: | 2.004 | 1.436 | 0.198 |
| Results: | POS | POS | POS |
| (POS: POSITIVE result; NEG: NEGATIVE result) | | | |

The serum analysed contains anti-HCV antibodies of type IgG and isotypes IgG1 and IgG3.

*Example 9. Patients undergoing haemodialysis with potential risk of occult HCV infection*

*Determining the cut-off value:*

The samples from patients undergoing haemodialysis with occult HCV infection are obtained following the method described in Example 1. The sample is then analysed to detect the presence of anti-HCV using ELISA with the method object of the invention. To do this, the reaction matrix is prepared following the description of example 3 using the reagent of example 2. The sample (plasma in this case) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

- The negative controls provide mean values of 0.057, 0.063 and 0.068 absorbance units at 405/620 nm; the mean value for the 3 negative controls is 0.062 absorbance units at 405/620 and the calculated standard deviation is 0.0057.
- The mean value for the positive control is 0.715 absorbance units at 405/620 nm.
- The cut-off value between the NEGATIVE and POSITIVE results is determined (test cut-off value) as the addition of the mean value of the 3 negative controls plus five times the standard deviation for the mean value.

The cut-off value for the test is 0.091 absorbance units at 405/620 nm.

Patient No. 10 (Patient No. 3 in the Priority Application) A patient (male) who due to his chronic kidney disease has been receiving haemodialysis for over 12 months. He receives analytical controls regularly and shows abnormal values of hepatic enzymes. - The plasma from Patient No. 3 provides absorbance values of 0.163 and 0.143 units at 405/620 nm; the mean value for the sample from Patient No. 3 is 0.153 absorbance units at 405/620 nm. The mean value for the sample from Patient No. 3 is greater to the cut-off value for the test, and is therefore assigned a POSITIVE result: the plasma analysed contains anti-HCV antibodies.

Patient No. 11 (Patient No. 4 in the Priority Application) A patient (male) who due to his chronic kidney disease (CKD) has been receiving haemodialysis for over 12 months. He receives blood transfusions due to his CKD-related anaemia. He presents abnormal values for liver enzymes in a laboratory test. - The plasma from Patient No. 4 provides absorbance values of 0.092 and 0.094 units at 405/620 nm; the mean value for the sample from Patient No. 4 is 0.093 absorbance units at 405/620 nm. The mean value for the sample from Patient No. 4 is greater to the cut-off value for the test, and is therefore assigned a POSITIVE result: the plasma analysed contains anti-HCV antibodies.

*Example 10: Other populations with potential risk of occult infection by HCV*

*Determining the cut-off value:*

The samples from people with potential risk of occult HCV infection (e.g., relatives of patients with occult HCV infection) are obtained following the method described in Example 1. The sample is then analysed to detect the presence of anti-HCV using ELISA with the method object of the invention. To do this, the reaction matrix is prepared following the description of example 3 using the reagent of example 2. The sample (serum in this case) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

- The negative controls provide mean values of 0.046, 0.050 and 0.047 absorbance units at 405/620 nm; the mean value for the 3 negative controls is 0.048 absorbance units at 405/620 and the calculated standard deviation is 0.0025.
- The mean value for the positive control is 1.466 absorbance units at 405/620 nm.
- The cut-off value between the NEGATIVE and POSITIVE results is determined (test cut-off value) as the addition of the mean value of the 3 negative controls plus five times the standard deviation for the mean value.
- The cut-off value for the test is 0.060 absorbance units at 405/620 nm.

Patient No. 12 (Patient No. 5 in the Priority Application) Relative (sister) of patient (male) diagnosed with genotype 1b occult HCV infection and mild chronic lobular hepatitis mild, who presents at the clinics for medical examination and laboratory tests.

- The serum sample from Patient No. 5 provides absorbance values of 0.169 and 0.173 units at 405/620 nm; the mean value for sample No. 5 is 0.171 absorbance units at 405/620 nm. The mean value for the sample from Patient No. 5 is greater than the cut-off value for the test, and is therefore assigned a POSITIVE result: the serum analysed contains anti-HCV antibodies.

Patient No. 13 (Patient No. 6 in the Priority Application) Relative (spouse) of patient (male) diagnosed with genotype 1 occult HCV infection, who presents at the clinics for medical examination and laboratory tests.

- The serum sample from Patient No. 6 provides absorbance values of 0.111 and 0.112 units at 405/620 nm; the mean value for sample No. 6 is 0.112 absorbance units at 405/620 nm. The mean value for the sample from Patient No. 6 is greater than the cut-off value for the test, and is therefore assigned a POSITIVE result: the serum analysed contains anti-HCV antibodies.

*Example 10bis: Other populations with potential risk of occult infection by HCV*

*Patient No. 14:*

Relative (daughter) of patient (female) diagnosed with cirrhosis of the liver due to classic HCV infection (anti-HCV and HCV-RNA detected in serum using commercial methods), who presents at the clinics for medical examination and analytical tests. Has normal values for liver enzymes and gives non-conclusive result for classic HCV infection (undetermined anti-HCV and HCV-RNA not detected in serum using commercial methods). She is diagnosed as an anti-HCV carrier. Liver biopsy shows mild steatosis and the presence of genotype 1 HCV-RNA, and she is therefore diagnosed with HCV infection.

- The serum sample from Patient No. 14-1 provides absorbance values of 0.117 and 0.117 units at 405/620 nm; the mean value for sample No. 14-1 is 0,117 absorbance units at 405/620 nm. The mean value for the sample from Patient No. 14-1 is greater to the cut-off value for the test, and is therefore assigned a POSITIVE result: the serum analysed contains anti-HCV antibodies.

The determination of anti-HCV is repeated on a subsequent sample of serum and gives a negative using commercial methods.

- The serum sample from Patient No. 14-2 provides absorbance values of 0.099 and 0.099 units at 405/620 nm; the mean value for sample No. 14-2 is 0,099 absorbance units at 405/620 nm. The mean value for the sample from Patient No. 14-2 is greater to the cut-off value for the test, and is therefore assigned a POSITIVE result: the serum analysed contains anti-HCV antibodies. She is diagnosed with occult HCV infection.

*Example 11. Patients with HCV infection with a genotype other than 1*

*Determining the cut-off value:*

The samples from patients diagnosed with HCV infection are obtained following the method described in Example 1. The sample is then analysed to detect the presence of anti-HCV using ELISA with the method object of the invention. To do this, the reaction matrix is prepared following the description of example 3 using the reagent of example 2.
The sample (in this case serum) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

- The negative controls provide mean values of 0.070, 0.069 and 0.074 absorbance units at 405/620 nm; the mean value for the 3 negative controls is 0.071 absorbance units at 405/620 and the calculated standard deviation is 0.004.
- The mean value for the positive control is 1.534 absorbance units at 405/620 nm.
- The cut-off value between the NEGATIVE and POSITIVE results is determined (test cut-off value) as the addition of the mean value of the 3 negative controls plus five times the standard deviation for the mean value.
- The cut-off value for the test is 0.091 absorbance units at 405/620 nm.

*Patient No. 15:*

Patient (female) who presents at the clinics due to abnormal values of hepatic enzymes. Classic HCV infection is diagnosed (anti-HCV and HCV-RNA detectable in serum using commercial methods). The liver biopsy proves the presence of genotype 3a HCV-RNA and liver lesion (active chronic hepatitis without liver fibrosis).

- The serum from Patient No. 15 provides absorbance values of 1.539 and 1.510 units at 405/620 nm; the mean value for the serum from Patient No. 15 is 1.525 absorbance units at 405/620 nm. The mean value for the sample from Patient No. 15 is greater than the cut-off value for the test, and is therefore assigned a POSITIVE result: the serum analysed contains anti-HCV antibodies.

*Patient No. 16:*

Patient (male) who presents at the clinics due to abnormal values of hepatic enzymes. Classic HCV infection is diagnosed (anti-HCV and HCV-RNA detectable in serum using commercial methods). The liver biopsy proves the presence of genotype 4 HCV-RNA and liver lesion (active chronic hepatitis with stage 1 liver fibrosis).

- The serum from Patient No. 16 provides absorbance values of 1.046 and 1.073 units at 405/620 nm; the mean value for the serum from Patient No. 16 is 1.060 absorbance units at 405/620 nm. The mean value for the sample from Patient No. 16 is greater than the cut-off value for the test, and is therefore assigned a POSITIVE result: the serum analysed contains anti-HCV antibodies.

Example 12. Immunoassay using as an antigen a peptide containing the sequence corresponding to SEQ.ID.NO.:3.

*Determining the cut-off value:*

The samples from patients diagnosed with HCV infection are obtained following the method described in Example 1. The sample is then analysed to detect the presence of anti-HCV using ELISA with the method object of the invention. To do this, prepare the reaction matrix as described in Example 3, using the reagent corresponding to SEQ.ID.NO.:3. The sample (in this case serum) is pre-treated as described in Example 4 and the immunochemistry reaction is performed as in Example 5, and the presence of anti-HCV is determined using ELISA following the methods described above in Examples 6 and 7. The determination is performed in duplicate using as controls the samples of 3 healthy people (negative controls) and 1 patient showing anti-HCV positive results by commercial ELISA (positive control) obtained and processed as described in Examples 1, 4 and 5. The following results are obtained when measuring the reaction:

- The negative controls provide mean values of 0.051, 0.059 and 0.058 absorbance units at 405/620 nm; the mean value for the 3 negative controls is 0.056 absorbance units at 405/620 and the calculated standard deviation is 0.004.
- The mean value for the positive control is 0.813 absorbance units at 405/620 nm.
- The cut-off value between the NEGATIVE and POSITIVE results is determined (test cut-off value) as the addition of the mean value of the 3 negative controls plus five times the standard deviation for the mean value.
- The cut-off value for the test is 0.076 absorbance units at 405/620 nm.

*Patient No. 17:*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and reactive changes are detected in the liver (minimum lesion). The serum from patient No. 17 gives a NEGATIVE result when the determination of anti-core HCV IgG antibodies is performed using the reagent described in Example 2.

- The serum from Patient No. 17 provides absorbance values of 0.084 and 0.092 units at 405/620 nm; the mean value for the serum from Patient No. 17 is 0.088 absorbance units at 405/620 nm. The mean value for the sample from Patient No. 17 is greater than the cut-off value for the test, and is therefore assigned a POSITIVE result: the serum analysed contains anti-HCV antibodies.

*Patient No. 18:*

Patient (male) who presents at the clinics due to abnormal values of hepatic enzymes. Known causes of liver disease are ruled out, including classic HCV infection (anti-HCV and HCV-RNA not detected in serum by commercial methods). The liver biopsy proves the presence of genotype 1b HCV-RNA, and therefore occult HCV infection is diagnosed and a liver lesion is detected (active chronic hepatitis with stage 4 liver fibrosis). The serum from patient No. 18 gives a POSITIVE result when the determination of anti-core HCV IgG antibodies is performed using the reagent described in Example 2.

- The serum from Patient No. 18 provides absorbance values of 0.531 and 0.486 units at 405/620 nm; the mean value for the serum from Patient No. 18 is 0.509 absorbance units at 405/620 nm. The mean value for the sample from Patient No. 18 is greater than the cut-off value for the test, and is therefore assigned a POSITIVE result: the serum analysed contains anti-HCV antibodies.

*Patient No. 19:*

Patient (female) who presents at the clinics due to abnormal values of hepatic enzymes. Classic HCV infection is diagnosed (anti-HCV and HCV-RNA detectable in serum using commercial methods). The liver biopsy proves the presence of genotype 3a HCV-RNA and liver lesion (active chronic hepatitis with stage 1 liver fibrosis).

- The serum from Patient No. 19 provides absorbance values of 0.929 and 1.000 units at 405/620 nm; the mean value for the serum from Patient No. 19 is 0.965 absorbance units at 405/620 nm. The mean value for the sample from Patient No. 19 is greater than the cut-off value for the test, and is therefore assigned a POSITIVE result: the serum analysed contains anti-HCV antibodies.

**Claims**

1. A method for the detection of occult hepatitis C, **characterised in that** it comprises performing a screening immunoassay comprising the following steps:

   a) incubating a solid-phase matrix with a HCV reagent, wherein the HCV reagent is a synthetic peptide of the HCV core protein consisting of SEQ.ID.NO:1,
   b) pre-treating the serum or plasma sample by a dilution and an incubation the purpose of which is to adsorb the sample components in order to eliminate the non-specific antigen-antibody bonds,
   c) adding the pre-treated sample obtained in step b) to the solid-phase matrix obtained in step a) and incubating the same for the antigen-antibody immunochemistry reaction,
   d) washout and reaction detection,
   e) washout and reaction development, and

f) reaction measurement.

2. The method according to claim 1, **characterised in that** the dilution defined in step b) is a dilution 1:10.

3. The method according to claim 1 or 2, **characterised in that** the incubation defined in step b) is at 37°C for 1 hour.

4. The method according to claim 1, **characterised in that** said screening immunoassay, as well as using the peptide consisting of SEQ.ID.NO:1 defined in step a), uses one or two of the peptides consisting of sequences: SEQ.ID.NO:2 and SEQ.ID.NO:3.

5. The method according to any one of the preceding claims 1 to 4, **characterised in that** said screening immunoassay is chosen from ELISA, a radioimmunoassay, a fluorescent immunoassay, a chemiluminescent immunoassay and a bioluminescent immunoassay.

6. The method according to any one of the preceding claims 1 to 4, **characterised in that** said samples are serum or plasma samples from peripheral blood or from organs, from fluids produced or secreted by isolated cells, or from the secretions of tissues or organs or from blood or fluids from organs.

7. The method according to any one of the preceding claims 1 to 6, **characterised in that** it is an ELISA.

8. The method according to claim 5, **characterised in that** said peptide has a sequence consisting of SEQ.ID.NO:1.

9. The method according to any one of claims 1 to 7, **characterised in that** it is an ELISA that uses as an antigen the peptide consisting of SEQ.ID.NO:1.

10. The method according to one of claims 1 to 9, **characterised in that** said screening immunoassay uses an antiserum marked with an isotopic tracer.

11. The method according to one of claims 1 to 10, **characterised in that** said screening immunoassay uses a human anti-IgG antiserum conjugated with a non-isotopic tracer.

12. The method according to claim 11, **characterised in that** said non-isotopic marker is chosen from a fluorophore, a chromophore, an enzyme or any other molecule that produces a suitable molecule for immunoassay.

13. The method according to claim 12, **characterised in that** said non-isotopic marker is the enzyme horseradish peroxidase, anti-IgG-HRP.

14. The method according to any one of the preceding claims 1 to 7, **characterised in that** it is an ELISA that uses as an antigen the peptide consisting of SEQ.ID.NO.:1 and because it uses human anti-IgG antiserum with the horseradish peroxidase enzyme, anti-IgG-HRP.

15. A kit for the implementation of the method for the detection of occult hepatitis C infection described in claims 1-14, **characterised in that** it comprises the following components:

    i) a solid-phase matrix,
    ii) a HCV reagent, wherein the HCV reagent is a synthetic peptide of the HCV core protein consisting of SEQ.ID.NO: 1, and one or two of the peptides consisting of sequences: SEQ.ID.NO:2 and SEQ.ID.NO:3,
    iii) a blocking agent,
    iv) a washout solution,
    v) a conjugate solution
    vi) a developing solution, and
    vii) a reaction stopper solution.

16. The kit according to claim 15, **characterised in that** the conjugated solution is a human anti-IgG antiserum with the horseradish peroxidase enzyme, anti-IgG-HRP.

17. The kit according to one of claims 15-16, **characterised in that** it comprises the following components:

i) a solid-phase matrix consisting of a flat bottom, sterile, 96-well EIA polystyrene microtitre plate, without caps,

ii) a HCV reagent, wherein the HCV reagent is a synthetic peptide of the HCV core protein consisting of SEQ.ID.NO: 1, and one or two of the peptides consisting of sequences: SEQ.ID.NO:2 and SEQ.ID.NO:3,

iii) a blocking agent consisting of 0.1 M buffered sodium and potassium phosphate solution at pH 7.4, supplemented with 10% of foetal bovine serum with 0.05% of polyoxyethylene-(20)-sorbitan monolaurate,

iv) a washout solution consisting of 0.1 M sodium phosphate buffer at pH 7.4 to which 0.05% of polyoxyethylene-(20)-sorbitan monolaurate,

v) a conjugate solution consisting of a polyclonal anti-human immunoglobulin G IgG, antiserum obtained from rabbits by immunisation with IgG isolated from normal human sera and conjugated with the horseradish peroxidase enzyme, anti-IgG-HRP,

vi) a developing solution consisting of 2,2'-azinobis-(3-ethylbenzothiazoline-6-sulphonic acid) diammonium salt as a developing solution, and

vii) a reaction stopper solution consisting of 1% n-sodium dodecyl sulphate.

## Patentansprüche

1. Verfahren zum Nachweis okkulter Hepatitis C, **dadurch gekennzeichnet, dass** es das Durchführen eines Screening-Immunoassays umfasst, welcher die folgenden Schritte umfasst:

   a) Inkubieren einer Festphasenmatrix mit einem HCV-Reagens, wobei es sich bei dem HCV-Reagens um ein synthetisches Peptid des HCV-Kernproteins, das aus SEQ ID NO: 1 besteht, handelt,
   b) Vorbehandeln der Serum- oder Plasmaprobe durch Verdünnung und Inkubation, deren Zweck darin besteht, die Komponenten der Probe zu adsorbieren, um die unspezifischen Antigen-Antikörper-Bindungen zu eliminieren,
   c) Zugeben der vorbehandelten, in Schritt b) erhaltenen Probe zu der in Schritt a) erhaltenen Festphasenmatrix und Inkubieren derselben für die Antigen-Antikörper-Immunchemiereaktion,
   d) Auswaschen und Nachweis der Reaktion,
   e) Auswaschen und Entwicklung der Reaktion und
   f) Messung der Reaktion.

2. Verfahren gemäß Anspruch 1, **dadurch gekennzeichnet, dass** es sich bei der in Schritt b) definierten Verdünnung um eine 1:10-Verdünnung handelt.

3. Verfahren gemäß Anspruch 1 oder 2, **dadurch gekennzeichnet, dass** die in Schritt b) definierte Inkubation bei 37°C 1 Stunde lang stattfindet.

4. Verfahren gemäß Anspruch 1, **dadurch gekennzeichnet, dass** der besagte Screening-Immunoassay ebenso, wie er das aus SEQ ID NO: 1 bestehende, in Schritt a) definierte Peptid einsetzt, eines oder zwei der aus den Sequenzen SEQ ID NO: 2 und SEQ ID NO: 3 bestehenden Peptide einsetzt.

5. Verfahren gemäß einem der vorangehenden Ansprüche 1 bis 4, **dadurch gekennzeichnet, dass** der besagte Screening-Immunoassay aus ELISA, einem Radioimmunoassay, einem Fluoreszenz-Immunoassay, einem Chemolumineszenz-Immunoassay und einem Biolumineszenz-Immunoassay ausgewählt ist.

6. Verfahren gemäß einem der vorangehenden Ansprüche 1 bis 4, **dadurch gekennzeichnet, dass** die besagten Proben Serum- oder Plasmaproben aus peripherem Blut oder von Organen, aus Flüssigkeiten, die von isolierten Zellen produziert oder sezerniert werden, oder aus den Sekretionen von Geweben oder Organen oder von Blut oder Flüssigkeiten von Organen sind.

7. Verfahren gemäß einem der vorangehenden Ansprüche 1 bis 6, **dadurch gekennzeichnet, dass** es sich um einen ELISA handelt.

8. Verfahren gemäß Anspruch 5, **dadurch gekennzeichnet, dass** das besagte Peptid eine Sequenz, die aus SEQ ID NO: 1 besteht, aufweist.

9. Verfahren gemäß einem der Ansprüche 1 bis 7, **dadurch gekennzeichnet, dass** es sich um einen ELISA handelt, der das aus SEQ ID NO: 1 bestehende Peptid als Antigen verwendet.

**10.** Verfahren gemäß einem der Ansprüche 1 bis 9, **dadurch gekennzeichnet, dass** der besagte Screening-Immunoassay ein Antiserum verwendet, das mit einem Tracer-Isotop markiert ist.

**11.** Verfahren gemäß einem der Ansprüche 1 bis 10, **dadurch gekennzeichnet, dass** der besagte Screening-Immunoassay ein humanes Anti-IgG-Antiserum verwendet, das mit einem Tracer, bei dem es sich nicht um ein Isotop handelt, konjugiert ist.

**12.** Verfahren gemäß Anspruch 11, **dadurch gekennzeichnet, dass** der besagte Marker, bei dem es sich nicht um ein Isotop handelt, aus einem Luminophor, einem Chromophor, einem Enzym oder jedem anderen Molekül, das ein geeignetes Molekül für einen Immunoassay ergibt, ausgewählt ist.

**13.** Verfahren gemäß Anspruch 12, **dadurch gekennzeichnet, dass** der besagte Marker, bei dem es sich nicht um ein Isotop handelt, das Enzym Meerrettichperoxidase, Anti-IgG-HRP, ist.

**14.** Verfahren gemäß einem der vorangehenden Ansprüche 1 bis 7, **dadurch gekennzeichnet, dass** es sich um einen ELISA handelt, der das aus SEQ ID NO: 1 bestehende Peptid als Antigen verwendet und weil er humanes Anti-IgG-Antiserum mit dem Enzym Meerrettichperoxidase, Anti-IgG-HRP, verwendet.

**15.** Kit zur Anwendung des in den Ansprüchen 1-14 beschriebenen Verfahrens zum Nachweis okkulter Hepatitis-C-Infektion, **dadurch gekennzeichnet, dass** es die folgenden Komponenten umfasst:

    i) eine Festphasenmatrix,
    ii) ein HCV-Reagens, wobei es sich bei dem HCV-Reagens um ein synthetisches Peptid des HCV-Kernproteins, das aus SEQ ID NO: 1 besteht, und eines oder zwei der aus den Sequenzen SEQ ID NO: 2 und SEQ ID NO: 3 bestehenden Peptide handelt,
    iii) ein Blockierungsmittel,
    iv) eine Auswaschlösung,
    v) eine Konjugatlösung,
    vi) eine Entwicklerlösung und
    vii) eine Reaktions-Stopperlösung.

**16.** Kit gemäß Anspruch 15, **dadurch gekennzeichnet, dass** es sich bei der Konjugatlösung um ein humanes Anti-IgG-Antiserum mit dem Enzym Meerrettichperoxidase, Anti-IgG-HRP, handelt.

**17.** Kit gemäß einem der Ansprüche 15-16, **dadurch gekennzeichnet, dass** es die folgenden Komponenten umfasst:

    i) eine Festphasenmatrix, bestehend aus einer sterilen 96-Loch-EIA-Polystyrolmikrotiterplatte mit flachem Boden ohne Kappen,
    ii) ein HCV-Reagens, wobei es sich bei dem HCV-Reagens um ein synthetisches Peptid des HCV-Kernproteins, das aus SEQ ID NO:1 besteht, und eines oder zwei der aus den Sequenzen SEQ ID NO:2 und SEQ ID NO:3 bestehenden Peptide handelt,
    iii) ein Blockierungsmittel, bestehend aus 0,1 M gepufferter Natrium- und Kaliumphosphatlösung bei pH 7,4, ergänzt mit 10 % fetalem Kälberserum mit 0,05 % Polyoxyethylen(20)-sorbitan-monolaurat,
    iv) eine Auswaschlösung, bestehend aus 0,1 M Natriumphosphatpuffer bei pH 7,4, zu der 0,05 % Polyoxyethylen(20)-sorbitan-monolaurat,
    v) eine Konjugatlösung, bestehend aus einem polyklonalem, anti-humanem Immunoglobulin G IgG, Antiserum, das von Kaninchen mittels Immunisierung mit IgG, das von normalen humanen Seren isoliert wurde, gewonnen und mit dem Enzym Meerrettichperoxidase konjugiert wurde, Anti-IgG-HRP,
    vi) eine Entwicklerlösung, bestehend aus 2,2'-Azinobis-(3-ethylbenzothiazolin-6-sulfonsäure)-diammoniumsalz als Entwicklerlösung und
    vii) eine Reaktions-Stopperlösung, bestehend aus 1 % n-Natriumdodecylsulfat.

## Revendications

**1.** Procédé pour la détection de l'hépatite C occulte, **caractérisé en ce qu'**il comprend la réalisation d'un dosage immunologique de dépistage comprenant les étapes suivantes :

a) l'incubation d'une matrice en phase solide avec un réactif du VHC, où le réactif du VHC est un peptide synthétique de la protéine de nucléocapside du VHC consistant en SEQ ID NO:1,

b) le prétraitement d'un échantillon de sérum ou de plasma par une dilution et une incubation, l'objectif de celui-ci étant d'adsorber les composants de l'échantillon afin d'éliminer les liaisons antigène-anticorps non spécifiques,

c) l'ajout de l'échantillon prétraité obtenu à l'étape b) à la matrice en phase solide obtenue à l'étape a) et l'incubation de ceux-ci pour la réaction d'immunochimie antigène-anticorps,

d) un lavage et la détection de la réaction,

e) un lavage et le développement de la réaction, et

f) la mesure de la réaction.

2. Procédé selon la revendication 1, **caractérisé en ce que** la dilution définie à l'étape b) est une dilution à 1:10.

3. Procédé selon la revendication 1 ou 2, **caractérisé en ce que** l'incubation définie à l'étape b) est réalisée à 37 °C pendant 1 heure.

4. Procédé selon la revendication 1, **caractérisé en ce que** ledit dosage immunologique de dépistage, ainsi que l'utilisation du peptide consistant en SEQ ID NO: 1 défini à l'étape a), utilise un ou deux des peptides consistant en les séquences : SEQ ID NO: 2 et SEQ ID NO: 3.

5. Procédé selon l'une quelconque des revendications 1 à 4 précédentes, **caractérisé en ce que** ledit dosage immunologique de dépistage est choisi parmi un ELISA, un dosage radioimmunologique, un dosage immunologique par fluorescence, un dosage immunologique par chimiluminescence et un dosage immunologique par bioluminescence.

6. Procédé selon l'une quelconque des revendications 1 à 4 précédentes, **caractérisé en ce que** lesdits échantillons sont des échantillons de sérum ou de plasma issus du sang périphérique ou d'organes, de liquides produits ou sécrétés par des cellules isolées, ou de sécrétions de tissus ou d'organes ou de sang ou de liquides d'organes.

7. Procédé selon l'une quelconque des revendications 1 à 6 précédentes, **caractérisé en ce que** c'est un ELISA.

8. Procédé selon la revendication 5, **caractérisé en ce que** ledit peptide possède une séquence consistant en SEQ ID NO: 1.

9. Procédé selon l'une quelconque des revendications 1 à 7, **caractérisé en ce que** c'est un ELISA qui utilise, en tant qu'antigène, le peptide consistant en SEQ ID NO: 1.

10. Procédé selon l'une quelconque des revendications 1 à 9, **caractérisé en ce que** ledit dosage immunologique de dépistage utilise un antisérum marqué avec un traceur isotopique.

11. Procédé selon l'une quelconque des revendications 1 à 10, **caractérisé en ce que** ledit dosage immunologique de dépistage utilise un antisérum humain anti-IgG conjugué à un traceur non isotopique.

12. Procédé selon la revendication 11, **caractérisé en ce que** ledit marqueur non isotopique est choisi parmi un fluorophore, un chromophore, une enzyme ou une quelconque autre molécule qui produit une molécule appropriée pour un dosage immunologique.

13. Procédé selon la revendication 12, **caractérisé en ce que** ledit marqueur non isotopique est l'enzyme peroxydase du raifort, anti-IgG-HRP.

14. Procédé selon l'une quelconque des revendications 1 à 7 précédentes, **caractérisé en ce que** c'est un ELISA qui utilise, en tant qu'antigène, le peptide consistant en SEQ ID NO: 1, et parce qu'il utilise un antisérum humain anti-IgG avec l'enzyme peroxydase du raifort, anti-IgG-HRP.

15. Kit pour la mise en oeuvre du procédé de détection d'une infection par l'hépatite C occulte décrit dans les revendications 1 à 14, **caractérisé en ce qu'**il comprend les composants suivants :

i) une matrice en phase solide,

ii) un réactif du VHC, où le réactif du VHC est un peptide synthétique de la protéine de nucléocapside du VHC consistant en SEQ ID NO: 1, et un ou deux des peptides consistant en les séquences : SEQ ID NO: 2 et SEQ ID NO: 3,
iii) un agent de blocage,
iv) une solution de lavage,
v) une solution de conjugué,
vi) une solution de développement, et
vii) une solution d'arrêt de la réaction.

16. Kit selon la revendication 15, **caractérisé en ce que** la solution de conjugué est un antisérum humain anti-IgG avec l'enzyme peroxydase du raifort, anti-IgG-HRP.

17. Kit selon l'une quelconque des revendications 15 à 16, **caractérisé en ce qu'**il comprend les composants suivants :

i) une matrice en phase solide consistant en une plaque de microtitration en polystyrène EIA stérile, à 96 puits à fond plat, sans couvercle
ii) un réactif du VHC, où le réactif du VHC est un peptide synthétique de la protéine de nucléocapside du VHC consistant en SEQ ID NO: 1, et un ou deux des peptides consistant en les séquences : SEQ ID NO: 2 et SEQ ID NO: 3,
iii) un agent de blocage consistant en une solution de phosphate de sodium et potassium tamponnée 0,1 M à un pH 7,4, supplémentée avec 10 % de sérum bovin foetal avec 0,05 % de monolaurate de polyoxyéthylène-(20)-sorbitane,
iv) une solution de lavage consistant en un tampon phosphate de sodium 0,1 M à un pH 7,4 auquel 0,05 % de de monolaurate de polyoxyéthylène-(20)-sorbitane,
v) une solution de conjugué consistant en un antisérum polyclonal anti-immunoglobuline G (IgG) humaine, obtenu chez des lapins par une immunisation avec une IgG isolée à partir de sérums humains normaux, et conjuguée à l'enzyme peroxydase du raifort, anti-IgG-HRP,
vi) une solution de développement consistant en un sel de diammonium d'acide 2,2'-azinobis-(3-éthylbenzothioazoline-6-sulfonique) en tant que solution de développement, et
vii) une solution d'arrêt de la réaction consistant en du n-dodécylsulfate de sodium à 1 %.

```
            1                 11                21                31
Consensus   M S T N P K P Q R K T K R N T N R R P Q D V K F P Q G G Q I V G G V Y L L P R R
Subtype 1a  M S T N P K P Q R K T K R N T N R R P Q D V K F P G G G Q I V G G V Y L L P R R
Subtype 1b  M S T N P K P Q R K T K R N T N R R P Q D V K F P G G G Q I V G G V Y L L P R R
Subtype 2a  N S T N P K P Q R K T K R N T S R R P Q D V K F P G G G Q I V G G V Y L L P R R
Subtype 2b  M S T I P K P Q R K T K R N T N R R P Q D V K F P Q G G Q I V G G V Y L L P R R
Subtype 3a  M S T L P K P Q R K T K R N T I R R P Q D V K F P G G G Q I V G G V Y L L P R R
Subtype 4a  M S T N P K P Q R K T K R N T N R R P N D V K P P G G G Q I V G G V Y L L P R R
Subtype 5a  M S T N P K P Q R K T K R N T N R R P Q D V K F P G G G Q I V G G V Y L L P R R
Sybtype 6a  M S T L P K P Q R K T K R N T N R R P M D V R P P Q G G Q I V G G V Y L L P R K


            41           .     51                61                71
Consensus   G P R L G V R A T R K T S E R S Q P R G R R Q P I P K A R R P E G R S W A Q P G
Subtype 1a  G P R L G V R A T R K T S E R S Q P R G R R Q P I P K A R R P E G R T W A Q P G
Subtype 1b  G P R L G V R A T R K T S E R S Q P R G R R Q P I P K A R R P E G R A W A Q P G
Subtype 2a  G P R L G V R A T R K T S E R S Q P R G R R Q P I P K D R R S T Q K S W Q K P G
Subtype 2b  G P R L G V R A T R K T S E R S Q P R G R R Q P I P K D R R S T G K S W G K P C
Subtype 3a  G P R L G V R A T R K T S E R S Q P R G R R Q P I P K A R R S E G R S W A Q P G
Subtype 4a  G P R L G V R A T R K T S E R S Q P R G R R Q P I P K A R R P E G R S W A Q P G
Subtype 5a  G P R L G V R A T R K N S E R S Q P R G R R Q P I P K A R R P T G R S W G Q P G
Sybtype 6a  G P R L G V R A T R K T S E R S Q P R G R R Q P I P K A R Q P Q G R H W A Q P G


        .   81                91                101               111
Consensus   Y P W P L Y G N E G C G W A G W L L S P R G S R P S W G P T D P R R R S R N L G
Subtype 1a  Y P W P L Y G N E G C G W A G N L L S P R G S R P S W G P T D P R R R S R N L G
Subtype 1b  Y P W P L Y G N E G L G W A G W L L S P R G S R P S W G P T D P R R R S R N V G
Subtype 2a  Y P W P L Y G N E G L G W A G W L L S P R G S R P S W G P T D P R H R S R N V G
Subtype 2b  Y P W P L Y G N E G C G W A G W L L S P R G S R P T W G P N D P R H R S R N L G
Subtype 3a  Y P W P L Y G N E G C G W A G W L L S P R G S R P S W G P N D P R R R S R N L G
Subtype 4a  Y P W P L Y G N E G C G W A G W L L S P R G S R P S W G P N D P R Q R S R N L G
Subtype 5a  Y P W P L Y A N E G L G W A G W L L S P R S S R P N W G P N D P R R K S P N L G
Sybtype 6a  Y P W P L Y G S R G C G W A Q W L L S P R G S R P H W G P N D P R R R S R N L G
```

Figure 1

25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES 200303050

### Non-patent literature cited in the description

- *Morbidity and Mortality Weekly Report,* 2003, vol. 52 (RR-3), 1-13
- **KUO G et al.** *Science,* 1989, vol. 244, 362-364
- **ALTER HJ et al.** *Lancet,* 1989, vol. 2, 1006-1008
- **VAN DER POEL CL et al.** *Vox Sang,* 1992, vol. 62, 208-212
- **KLEINMAN S et al.** *Transfusion,* 1992, vol. 32, 805-813
- **OKAMOTO H et al.** *Hepatology,* 1992, vol. 15, 180-186
- **SÄLLBERG M et al.** *J Clin Microbiol,* 1992, vol. 30, 1989-1994
- **SÄLLBERG M et al.** *Immunol Lett,* 1992, vol. 33, 27-33
- **BRESTERS D et al.** *J Med Virol,* 1992, vol. 37, 187-191
- **SATO A et al.** *J Med Virol,* 1994, vol. 44, 88-91
- **SÄLLBERG M et al.** *J Med Virol,* 1994, vol. 43, 62-68
- **MONDELLI MU et al.** *J Clin Microbiol,* 1994, vol. 32, 2523-2527
- **CHEN M et al.** *Gastroenterology,* 1999, vol. 116, 135-143
- **TOYODA H et al.** *Am J Gastroenterol,* 1999, vol. 94, 2230-2236
- **LAPERCHE S et al.** *J Clin Microbiol,* 2005, vol. 43, 3877-383
- **NETSKI DM et al.** *Clin Infect Dis,* 2005, vol. 41, 667-675
- **MUERHOFF AS et al.** *J Med Virol,* 2008, vol. 80, 411-418
- **QUIROGA JA et al.** *Hepatology,* 1991, vol. 14, 38-43
- **QUIROGA JA et al.** *Clin Diag Lab Immunol,* 1994, vol. 1, 545-551
- **QUIROGA JA et al.** *J Infect Dis,* 1996, vol. 173, 300-305
- **MARTIN J et al.** *Cytokine,* 1998, vol. 10, 635-644
- **QUIROGA JA et al.** *J Clin Microbiol,* 2006, vol. 44, 4559-4560
- **QUIROGA JA et al.** *Clinical and Vaccine Immunology,* 2007, vol. 14 (10), 1302-1306
- **HARLOW D ; LANE D.** Antibodies: A laboratory manual. Cold Spring Harbor Laboratory, 1988, 592